# EUROPÄISCHE PATENTSCHRIFT

(11) **EP 3 936 855 B1**
(45) Veröffentlichungstag und Bekanntmachung des Hinweises auf die Patenterteilung: **08.03.2023**
(21) Anmeldenummer: 21183171.4
(22) Anmeldetag: 01.07.2021
(51) Int. Cl.: A61M 16/22, B63C 11/24, G01F 23/02

(54) **KOHLENSTOFFDIOXIDABSORBER FÜR EIN KREISLAUFATEMGERÄT**
CARBON DIOXIDE ABSORBER FOR A CIRCUIT RESPIRATOR
ABSORBEUR DE DIOXYDE DE CARBONE POUR UN RECYCLEUR

(30) Priorität: 07.07.2020 DE 102020117894
(43) Veröffentlichungstag der Anmeldung: 12.01.2022
(73) Patentinhaber: Dräger Safety AG & Co. KGaA, 23560 Lübeck (DE)
(72) Erfinder: Krüger, Lorenz, 23558 Lübeck (DE); Libicher, Christopher, 23558 Lübeck (DE); Weber, Tim, 23558 Lübeck (DE)
(74) Vertreter: Guthöhrlein, Gerhard

(56) Entgegenhaltungen:
- WO-A1-92/20404
- DE-B3-102013 018 588
- US-A- 4 108 172
- US-A1- 2014 345 610

## Beschreibung

Die Erfindung betrifft einen Kohlenstoffdioxidabsorber mit einem Einlass und einem Auslass, die mit einem Kreislaufatemgerät verbindbar sind und die durch einen Strömungskanal gasdicht miteinander verbunden sind, in dem ein Material angeordnet ist, das wenigstens einen Teil des in einem durch das Material geleiteten Atemgasstrom enthaltenen Kohlenstoffdioxid absorbiert. Derartige Kohlenstoffdioxidabsorber lassen sich sowohl in Atemschutzkreislaufgeräten, wie sie beispielsweise im Bergbaurettungswesen zum Einsatz kommen, oder in Kreislauftauchgeräte verwenden.

Wesentlich an den aus dem Stand der Technik bekannten Kreislaufatemgeräten ist, dass der Geräteträger unabhängig von der Umgebungsatmosphäre mit Sauerstoff versorgt wird, wobei das benötigte Atemgas im Gegensatz zu Pressluftatemgeräten innerhalb des Gerätes erzeugt wird. Nach der Veratmung des dem Geräteträger zugeleiteten Atemgases, wird dieses in einem geräteinternen Kreislauf aufbereitet bevor es dem geräteträger wieder zugeleitet wird, wobei dem ausgeatmeten Atemgas während der Aufbereitung Kohlenstoffdioxid entzogen und Sauerstoff zugeführt wird.

Kreislaufatemgeräte werden üblicherweise auf dem Rücken oder am Bauch getragen und sind über Atemschläuche mit einer Atemmaske verbunden, über die der Geräteträger ein- und ausatmet. Als Sauerstoffquelle wird entweder Drucksauerstoff oder Chemikaliensauerstoff mitgeführt. Die Entfernung des vom Geräteträger ausgeatmeten Kohlenstoffdioxids (CO₂) erfolgt in den bekannten Kreislaufatemgeräten mithilfe des sogenannten Atemkalks, der in Patronen oder Kartuschen mitgeführt wird, die als Einwegartikel oder wiederbefüllbar ausgeführt sein können. Als Atemkalk wird in der Regel eine granulatartige Mischung aus Kalziumhydroxid (Ca(OH)₂) und Natriumhydroxid (NaOH) oder aus Kaliumhydroxid (KOH) und Bariumhydroxid (Ba(OH)₂) verwendet.

Während das vom Geräteträger ausgeatmete Atemgas die Atemkalkschüttung innerhalb der Atemkalkpatrone durchströmt, wird das im Atemgas enthaltene Kohlenstoffdioxid absorbiert und es entstehen Wärme und Wasser. Üblicherweise verwendeter Atemkalk absorbiert 10-15 I Kohlenstoffdioxid je 100 g Schüttgut. Um einen einwandfreien, sicheren Betrieb eines Kreislaufatemgerätes zu gewährleisten, muss stets ausreichend Atemkalk, der in der Lage ist, Kohlenstoffdioxid zu absorbieren, in der mitgeführten Atemkalkpatrone enthalten sein.

Ein gattungsgemäßes Kreislaufatemgerät ist aus der DE 10 2011 014 267 B4 bekannt. Das beschriebene Kreislaufatemgerät verfügt über ein Gehäuse sowie Atemschläuche zur Zu- und Ableitung von Atemluft in und aus einer Atemmaske. Das vom Geräteträger ausgeatmete Atemgas wird durch eine Atemkalkpatrone geleitet, in der dem ausgeatmeten Atemgasstrom Kohlenstoffdioxid entzogen wird. Stromabwärts der Atemkalkpatrone wird dem Atemgas eine geringfügige Menge Sauerstoff aus einer Druckgasflaschen zugeführt und das so aufbereitete Atemgas dem Geräteträger schließlich über die Atemmaske wieder zugeleitet.

Im Weiteren ist aus der DE 10 2013 018 588 B3 eine Vorrichtung sowie ein Verfahren zur Bestimmung des Sättigungsgrades von Atemkalk bekannt. Die beschriebene Vorrichtung verfügt über eine Strahlungsquelle, die Strahlung mit wenigstens zwei unterschiedlichen Wellenlängen emittiert, über einen Sensor, über eine Datenverarbeitungseinheit und über einen Lichtleiter, der zumindest teilweise in einer mit Atemkalk gefüllten Atemkalkpatrone angeordnet ist. Die von der Strahlungsquelle emittierte Strahlung wird in den Lichtleiter und von hier in den innerhalb der der Atemkalkpatrone angeordneten Atemkalk eingekoppelt. Die von dem Atemkalk reflektierte Strahlung wird wieder in den Lichtleiter eingekoppelt und durch den Lichtleiter zu dem Sensor geleitet, wobei schließlich aus der Intensität der reflektierten Strahlung ein Sättigungsgrad des Atemkalks ermittelt wird. Mit der beschriebenen Vorrichtung ist es somit möglich, den Sättigungsgrad bzw. das noch bestehende Bindungsvermögen des Atemkalks zu bestimmen.

Während der Nutzung von Kreislaufatemgeräten besteht stets ein Risiko darin, dass der Atemkalk gesättigt, also sein Vermögen, Kohlenstoffdioxid zu absorbieren, erschöpft ist, oder dass die erforderliche Atemkalkpatrone nicht oder zumindest nicht korrekt in das Kreislaufatemgerät eingesetzt ist. Aus diesem Grund besteht ein genereller Bedarf an technischen Lösungen, die den Geräteträger auf möglichst einfache und zuverlässige Lösung darüber informieren, ob und eventuell wie lange ein Kreislaufatemgerät einsatzfähig ist.

In diesem Zusammenhang ist das Kreislauftauchgerät Explorer der Firma Hollis bekannt, das über eine Anzeige verfügt, die den Geräteträger darüber informiert, ob sich ein Kohlenstoffdioxidabsorber im Gerät befindet. Sobald eine Atemkalkpatrone in das Gerät eingesetzt wird, drückt diese entgegen einer Federkraft auf einen Anzeigestift, der sich daraufhin nach außen bewegt und durch ein Fenster sichtbar ist. Auf diese Weise wird dem Geräteträger angezeigt, dass sich eine Atemkalkpatrone ordnungsgemäß eingesetzt im Gerät befindet. Allerdings erhält der Geräteträger aufgrund dieser Anzeige keine Information über den Füllstand bzw. das noch vorhandene Kohlenstoffdioxid-Absorptionsvermögen der eingesetzten Atemkalkpatrone.

Ausgehend von den aus dem Stand der Technik bekannten Lösungen sowie den zuvor geschilderten Problemen liegt der Erfindung die Aufgabe, einen Kohlenstoffdioxidabsorber anzugeben, bei dem einfach und zuverlässig der Füllstand des Atemkalks, der sich im Inneren befindet, erkennbar ist. Insbesondere soll eine visuelle Prüfung möglich sein, ohne dass der Kohlenstoffdioxidabsorber hierfür demontiert und/oder geöffnet werden muss. Die anzugebende technische Lösung sollte es somit einem Nutzer auf einfache, sichere und zuverlässige Weise ermöglichen, den Füllstand einer Atemkalkpatrone von außen visuell wahrzunehmen.

Im Weiteren sollte die anzugebende technische Lösung eine Möglichkeit schaffen, ohne Öffnen eines Kreislaufatemgeräts den Füllstand eines im Gerät montierten Kohlenstoffdioxidabsorbers visuell zu erfassen. Hierbei ist es stets von besonderer Bedeutung, dass es für einen Nutzer, insbesondere vor Inbetriebnahme eines Kreislaufatemgeräts, deutlich erkennbar ist, ob sich eine Atemkalkkartusche im Gerät befindet und ob eine eingesetzte Atemkalkkartusche ordnungsgemäß befüllt ist, um Kohlenstoffdioxid in dem erforderlichen Maß absorbieren zu können und einen sicherer Betrieb für einen Geräteträger zu gewährleisten.

Des Weiteren sollte eine entsprechende Anzeigeeinheit wartungsarm sein, einen ungestörten Betrieb über einen möglichst langen Zeitraum ermöglichen und über einen konstruktiv vergleichsweise einfachen Aufbau verfügen, sodass auch die Herstellkosten in einem ökonomisch sinnvollen Rahmen gehalten werden können. Auf vorteilhafte Weise sollte sich eine entsprechende Anzeige realisieren lassen, ohne dass hierfür zusätzliche Energie, insbesondere in Form von elektrischer Energie, benötigt wird.

Die zuvor genannte Aufgabe wird mit einem Kohlenstoffdioxidabsorber gemäß Anspruch 1 sowie einem Kreislaufatemgerät nach Anspruch 14 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft einen Kohlenstoffdioxidabsorber mit einem Einlass und einem Auslass, die mit einem Kreislaufatemgerät, insbesondere dessen Atemgaskreislauf, verbindbar sind und die durch einen Strömungskanal gasdicht miteinander verbunden sind, in dem ein Material angeordnet ist, das wenigstens einen Teil des in einem durch das Material geleiteten Atemgasstrom enthaltenen Kohlenstoffdioxids absorbiert. Erfindungsgemäß zeichnet sich der Kohlenstoffdioxidabsorber dadurch aus, dass der Strömungskanal wenigstens bereichsweise von einem Gehäuse umgeben ist, in dem ein Fensterelement angeordnet ist, durch das ein im Strömungskanal bewegbar angeordnetes Anzeigeelement von außerhalb des Gehäuses sichtbar ist und/oder durch das vom Anzeigeelement reflektierte Strahlung nach außen tritt, wobei ein Abstand zwischen dem Fensterelement und dem Anzeigeelement in Abhängigkeit der im Strömungskanal angeordneten Menge von kohlenstoffdioxidabsorbierendem Material variiert. Wesentlich ist somit die Kombination aus einem Fensterelement, das den Blick ins Innere des Gehäuses eines Kohlenstoffdioxidabsorbers mit dem darin angeordneten kohlenstoffdioxidabsorbieren Material freigibt, und einen im Inneren des Gehäuses angeordneten Anzeigeelement, dessen Position sich in Abhängigkeit der Füllmenge von kohlenstoffabsorbierendem Material, insbesondere Atemkalk, derart verändert, dass sich hierdurch auch ein Abstand zwischen dem Fensterelement und dem Anzeigeelement ebenfalls verändert. Die Bewegung des Anzeigeelementes im Inneren des Gehäuses des Kohlenstoffdioxidabsorbers erfolgt hierbei in Abhängigkeit des Füllstandes bzw. der Füllmenge von kohlenstoffabsorbierendem Material derart kontrolliert, dass bei einem Blick in das Fensterelement für den Geräteträger das Anzeigeelement und/oder eine von diesem reflektierte Strahlung derart sichtbar ist, dass er aus der Anordnung des Anzeigeelementes relativ zum Fensterelement und/oder anhand eines Anzeigeobjekts, beispielsweise einem speziellen Lichtobjekt, das durch vom Anzeigeelement ausgehende Strahlung verursacht wird, eine Information über den aktuellen Füllstand oder die Füllmenge von kohlenstoffdioxidabsorbierendem Material erhält. Wird ein erfindungsgemäß ausgeführter Kohlenstoffdioxidabsorber in ein Kreislaufatemgerät eingebaut, das seinerseits die Möglichkeit bietet, das Fensterelement von außen zu erkennen, ohne dass hierfür ein Gehäuse des Kreislaufatemgeräts geöffnet werden muss, kann ein Nutzer und/oder Geräteträger ferner sowohl erkennen, ob sich im Inneren des Kreislaufatemgerätes ein Kohlenstoffdioxidabsorber befindet als auch eine Information über den Füllstand im Kohlenstoffdioxidabsorber erhalten. Es ist somit wiederum nicht erforderlich, das Kreislaufatemgerät und/oder den Kohlenstoffdioxidabsorber zu öffnen oder zu demontieren.

Von besonderem Vorteil ist es, wenn das Anzeigeelement derart ausgeführt ist, dass dieses von außen durch das Fensterelement zu erkennen ist, ohne dass das Anzeigeelement selbst über eine Lichtquelle verfügt. Die Oberfläche ist daher vorzugsweise derart ausgeführt, dass auftreffende Strahlung in Richtung auf das Fensterelement reflektiert wird.

Ferner ist ein erfindungsgemäß ausgeführter Kohlenstoffdioxidabsorber bevorzugt in Form einer Patrone oder Kartusche ausgeführt, die über einen Einlass und einen Auslass verfügt, an denen der Atemgaskreislauf eines Kreislaufatemgerätes, entweder eines Atemschutzkreislaufgeräts, wie es etwa im Bergbaurettungswesen eingesetzt wird, oder eines Kreislauftauchgeräts, gasdicht anschließbar ist. Auf vorteilhafte Weise verfügt der Einlass und/oder der Einlass hierfür über geeignete Anschlusselemente, die ein schnelles und sicheres Verbinden einer Atemkalkpatrone oder -kartusche mit dem Atemgaskreislauf ermöglichen. Denkbar ist etwa die Herstellung einer Verbindung mit Hilfe eines Bajonett- oder Gewindeverschlusses.

Gemäß einer besonderen Ausführungsform der Erfindung ist vorgesehen, dass das Anzeigeelement auf einer innerhalb des Strömungskanals bewegbaren Druckeinheit angeordnet ist, die mittelbar oder unmittelbar am Absorbermaterial anliegt. Hierbei ist es denkbar, dass das Anzeigeelement auf oder an der Druckeinheit befestigt oder einteilig mit der Druckeinheit ausgebildet ist.

Die Druckeinheit ist derart bewegbar innerhalb des Strömungskanals mit dem darin befindlichen kohlenstoffdioxidabsorbierenden Material angeordnet, dass eine gezielte Bewegung dann erfolgt, wenn sich der Füllstand oder die Füllmenge des im Strömungskanal angeordneten kohlenstoffdioxidabsorbierenden Materials ändert. Vorzugsweise wird die Druckeinheit von einer Federkraft beaufschlagt, wobei die Federkraft derart auf die Druckeinheit einwirkt, dass die Druckeinheit in Richtung auf das kohlenstoffdioxidabsorbierende Material gedrückt oder sogar bewegt wird. Das Federelement ist in diesem Fall bevorzugt derart ausgebildet, dass nur eine leichte Federkraft in Richtung auf das im Inneren des Kohlenstoffdioxidabsorbers angeordnete Material ausgeübt wird und so das Material mit einer vergleichsweise geringen Kraft beaufschlagt wird. Aufgrund der auf die Druckeinheit einwirkenden Federkraft wird sie bei der Änderung des Füllstandes oder der Füllmenge des im Kohlenstoffdioxidabsorber angeordneten Materials, insbesondere bei Abnahme des Füllstandes oder der Füllmenge, bewegt und somit auch der Abstand zwischen dem Fensterelement und dem mittelbar oder unmittelbar mit der Druckeinheit verbundenen Anzeigeelement verändert.

Gemäß einer weiteren speziellen Ausführungsform verfügt die Druckeinheit wenigstens bereichsweise über ein Siebelement, das an dem kohlenstoffdioxidabsorbierenden Material anliegt. Weiterhin ist es denkbar, dass die Druckeinheit an ihrem äußeren Umfang, insbesondere mittelbar über ein an der Wand des Strömungskanals anliegendes Dichtelement, wenigstens bereichsweise an der Wand des Strömungskanals anliegt. Mit Hilfe der Druckeinheit wird das im Strömungskanal angeordnete Material somit bei Veränderung der Füllmenge bzw. des Füllstandes zusammengeschoben, sodass das Material auch weiterhin auf geeignete Weise von dem von Kohlenstoffdioxid zu befreienden Atemgas durchströmt wird.

In einer speziellen Weiterbildung der Erfindung ist auf dem Fensterelement wenigstens eine Markierung angeordnet. In diesem Zusammenhang ist es denkbar, dass eine derartige Markierung eine Messskala, eine lineare Skalierung, konzentrische Kreise und/oder ein Fadenkreuz aufweist. Die Markierung ermöglicht es dem Geräteträger auf vergleichsweise einfache Weise, den jeweiligen Füllstand oder die Füllmenge des im Inneren des Kohlenstoffdioxidabsorbers angeordneten Absorbermaterials abzulesen. Denkbar ist etwa, dass die Markierung ein Fadenkreuz oder eine Mehrzahl von konzentrischen Kreisen aufweist, die in Abhängigkeit des Abstandes des Anzeigeelements vom Fensterelement mehr oder weniger von einer Abbildung des Anzeigeelements überdeckt werden. Wesentlich für diese technische Lösung ist, dass die äußere Kontur des Anzeigeelements bzw. dessen Abbildung auf dem Fensterelement in Relation zu der Markierung gesetzt wird, sodass für den Geräteträger eine eindeutige und leicht zu erfassende Information in Bezug auf den Füllstand oder die Füllmenge des kohlenstoffdioxidabsorbierenden Materials ausgegeben wird.

In einer weiteren besonderen Ausführungsform der Erfindung weist das Anzeigeelement wenigstens ein opakes Material auf, sodass das Anzeigeelement nicht oder nur teilweise für auftreffende Strahlung transparent ist. Bei einem derartigen Material handelt es sich bevorzugt um einen Kunststoff, eine Mischung verschiedener Kunststoffe und/oder ein kristallines Material. Von Bedeutung für das zum Einsatz kommende opake Material ist, dass dieses für auftreffende Strahlung nicht vollständig transparent ist, sondern zumindest in einem Teil des Strahlenbereiches der Strahlung reflektiert. Opakes Material zeichnet sich hierbei generell dadurch aus, dass es für zumindest einen Teil der auftreffenden Strahlung streuende Eigenschaften aufweist.

In einer ganz besonderen Ausgestaltung der Erfindung ist ein Optikelement vorgesehen, durch das ein von außen in das Fensterelement eintretender Lichtstrahl von einer vom Fensterelement zum Anzeigeelement verlaufenden Mittenachse divergent weggelenkt wird, sodass die sich vom Fensterelement zum Anzeigeelement fortpflanzende Strahlung divergiert. Vorzugsweise handelt es sich bei dem Optikelement um eine Linse und/oder ein Prisma, durch das auftreffende Strahlung auf geeignete Weise umgelenkt und/oder geformt wird. Generell ist es denkbar, dass das verwendete Optikelements zwischen Fensterelement und dem Anzeigeelement angeordnet ist oder in das Fensterelement integriert ist. In diesem Zusammenhang sieht eine ganz spezielle Weiterbildung der Erfindung vor, dass das Fensterelement zumindest bereichsweise in Form eines Optikelementes ausgebildet ist, dass die in das Fensterelement von außen aus einer Umgebung eintretende Strahlung von einer vom Fensterelement zum Anzeigeelement verlaufenden Mittenachse divergent weglenkt. Bevorzugt ist das Fensterelement somit derart ausgeführt, dass auftreffende Lichtstrahlen an den jeweiligen Kanten, die Übergänge zwischen unterschiedlichen Medien, insbesondere zwischen einem festen Medium und Luft, darstellen, also insbesondere am Übergang zwischen dem Fensterelement und dem Innenraum des Kohlenstoffdioxidabsorbers derart gebrochen werden, dass die sich im Innenraum fortpflanzende Strahlung divergiert. Wesentlich ist, dass die Strahlung sowohl beim Eintritt in das Fensterelement als auch beim Austritt aus diesem gebrochen wird. Je weiter das Anzeigeelement von der dem Anzeigeelement zugewandten Körperkante des Fensterelementes entfernt ist, also je größer der Abstand zwischen dem Fensterelement und dem Anzeigeelement ist, desto weniger Lichtstrahlen treffen auf das Anzeigeelement und werden von diesem reflektiert. Hieraus folgt, dass während des Betriebs eines Kreislaufatemgeräts und hierbei abnehmendem Füllstand des verwendeten Absorbermaterials die durch das Fensterelement wahrnehmbare Abbildung des Anzeigeelementes, immer kleiner wird, bis sie nicht mehr wahrgenommen werden kann, da der Abstand zwischen Anzeigeelement und dem Fensterelement zu groß geworden ist. Gemäß dieser speziellen Ausführungsform der Erfindung wird somit der Abstand zwischen dem Anzeigeelement und dem Fensterelement genutzt, um eine Abbildung des Anzeigeelements im Bereich des Fensterelementes zu erzeugen, wobei die Größe der Abbildung des Anzeigelements in Relation zum Füllstand des Kohlenstoffdioxidabsorbers gesetzt wird. In einer besonderen Ausgestaltung entsprechen unterschiedliche Füllstände jeweils verschiedenen Abbildungsdurchmessern, wobei vorzugsweise der Radius einer Abbildung des Anzeigeelements im Bereich des Fensterelements als Funktion des Abstands zwischen dem Fensterelement und dem Anzeigeelement berechenbar ist.

In einer besonderen Ausführungsform der Erfindung befindet sich in dem vom aufzubereitenden Atemgas durchströmten Strömungskanal des Kohlenstoffdioxidabsorbers ein kohlenstoffabsorbierendes Material, das als Schüttgut ausgeführt ist. Vorzugsweise handelt es sich bei dem für die Absorption von Kohlenstoffdioxid verwendeten Material, also dem sogenannten Atemkalk, um eine Mischung aus Calciumhydroxid (Ca(OH)₂) und Natriumhydroxid (NaOH) oder aus Kaliumhydroxid (KOH) und Bariumhydroxid (Ba(OH)₂). Generell ist es denkbar, dass das Absorbermaterial in einer Einwegkartusche oder -patrone, die, nachdem das Absorptionsvermögen des Absorbermaterials aufgebraucht ist, verworfen wird, oder in einem wiederbefüllbaren Mehrwegbehälter im Kohlenstoffdioxidabsorbereit bereitgestellt wird. In diesem Zusammenhang ist es denkbar, dass der vom Gehäuse umschlossene Strömungskanal wenigstens eine verschließbare Füllöffnung aufweist, durch die das Absorbermaterial wenigstens zeitweise einfüllbar und/oder entnehmbar ist.

Im Übrigen wird die Erfindung mit einem Kreislaufatemgerät gelöst, das über einen Kohlenstoffdioxidabsorber verfügt, der gemäß zumindest einem der zuvor beschriebenen Ausführungsformen ausgebildet ist. Das Kreislaufatemgerät kann entweder als Atemschutzkreislaufgerät, etwa im Rettungswesen, insbesondere im Bergbaurettungswesen, oder als Kreislauftauchgerät ausgeführt sein. Wesentlich ist jeweils, dass der Kohlenstoffdioxidabsorber über seinen Einlass und den Auslass derart mit dem Atemgaskreislauf des Kreislaufatemgerätes verbunden ist, dass dem von einem Geräteträger ausgeatmeten Atemgas zumindest ein Teil des darin enthaltenen Kohlenstoffdioxids entzogen werden kann. Vorteilhaft verfügt das Kreislaufatemgerät, insbesondere im Bereich seines Gehäuses ebenfalls über wenigstens ein Fensterelement, das wiederrum den Blick auf das Fensterelement des Kohlenstoffdioxidabsorbers und somit zumindest mittelbar auf das Anzeigeelement freigibt. Auf diese Weise kann ein Geräteträger, insbesondere vor Inbetriebnahme des Geräts, den Füllstand des Kohlenstoffdioxidabsorbers ablesen, ohne hierfür das Kreislaufatemgerät öffnen zu müssen. Ferner ist es denkbar, dass ein erfindungsgemäß ausgeführtes Kreislaufatemgerät einer Einweg- oder einer Mehrwegkartusche oder -patrone als Kohlenstoffdioxidabsorber ausrüstbar ist.

Im Folgenden wird die Erfindung ohne Beschränkung des allgemeinen Erfindungsgedankens anhand von Ausführungsbeispielen unter Bezugnahme auf die Figuren näher erläutert. Dabei zeigen:
- Fig. 1:: Schematische Darstellung eines Kreislaufatemgeräts mit einem erfindungsgemäß ausgeführten Kohlenstoffdioxidabsorber;
- Fig. 2:: Schnittansicht eines erfindungsgemäß ausgeführten Kohlenstoffdioxidabsorbers sowie
- Fig. 3:: Schematische Darstellung des Wirkprinzips zur Erfassung des Füllstandes eines erfindungsgemäß ausgeführten Kohlenstoffdioxidabsorbers unter Ausnutzung einer Abstandsänderung zwischen einem Fensterelement und einem Anzeigeelement.

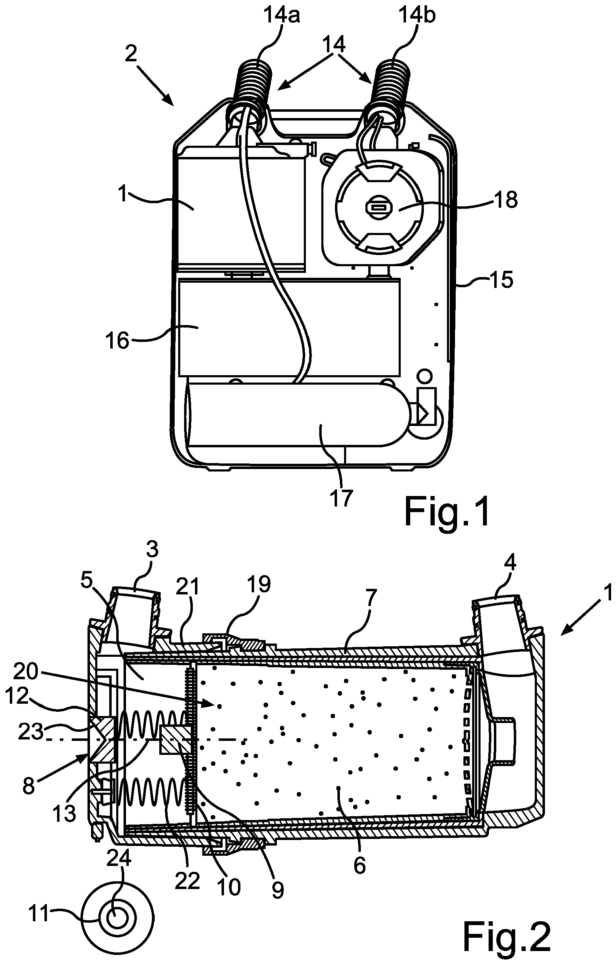

Fig. 1 zeigt ein Kreislaufatemgerät 2, in dem ein erfindungsgemäß ausgeführter Kohlenstoffdioxidabsorber 1 verbaut ist. Das Kreislaufatemgerät 2 verfügt über zwei Atemschläuche 14 zur Zu- und Ableitung von Atemgas in eine nicht dargestellte Atemmaske, die von einem Geräteträger getragen wird. Von den beiden Atemschläuchen 14 stellt der rechts angeordnete Atemschlauch den Einatemschlauch 14b, der links angeordnete Atemschlauch den Ausatemschlauch 14a dar.

Beide Atemschläuche 14 sind mit einem Gehäuse 15 des Kreislaufatemgerätes 2 verbunden. In dem Gehäuse 15 ist ein Kohlenstoffdioxidabsorber 1 zur wenigsten teilweisen Entfernung des während der Nutzung des Kreislaufatemgerätes 2 vom Geräteträger ausgeatmeten Kohlenstoffdioxids (CO₂) angeordnet. Unterhalb des Kohlenstoffdioxidabsorbers 1 ist ein Atembeutel 16 an dem Gehäuse 15 befestigt. Weiterhin ist innerhalb des Gehäuses 15 eine Sauerstoffquelle 17, die in der dargestellten Ausführungsform durch eine Sauerstoffdruckgasflasche gebildet wird, angeordnet. Da bei der Absorption von Kohlenstoffdioxid (CO₂) Wärme entsteht, verfügt das in Fig. 1 gezeigte Kreislaufatemgerät 2 über einen Atemgaskühler 18, der die bei der Kohlenstoffdioxidabsorption anfallende Wärme zumindest teilweise aus dem Atemgaskreislauf abführt. Während des Betriebs des in Fig. 1 gezeigten Kreislaufatemgerätes 2 gelangt das vom Geräteträger ausgeatmete Atemgas über den Ausatemschlauch 14a zunächst in den Kohlenstoffdioxidabsorber 1, wo es zumindest teilweise von dem im Atemgas enthaltenen Kohlenstoffdioxid (CO₂) befreit wird, indem das hier angeordnete kohlenstoffdioxidabsorbierende Material 6 einen Teil des Kohlenstoffdioxids unter Bildung von Wärme und Wasser absorbiert. Anschließend wird das Atemgas in den Atembeutel 16 geleitet, in dem der vom Geräteträger verbrauchte Sauerstoff mit Sauerstoff aus der als Sauerstoffquelle 17 mitgeführten Sauerstoffdruckgasflasche ersetzt wird. Das von überschüssigem Kohlenstoffdioxid befreite und mit Sauerstoff angereicherte Atemgas durchströmt daraufhin den Atemgaskühler 18, in dem ein Teil der bei der Absorption von Kohlenstoffdioxid freigewordenen Reaktionswärme aus dem Atemgaskreislauf abgeführt wird. Über den Einatemschlauch 14b wird das wie zuvor beschrieben regenerierte Atemgas in eine nicht dargestellte Atemmaske geleitet, über die es vom Geräteträger eingeatmet wird.

Fig. 2 zeigt einen erfindungsgemäß ausgeführten Kohlenstoffdioxidabsorber 1. Derartige Kohlenstoffdioxidabsorber 1 können als Einweg- oder Mehrwegkartusche oder -patrone ausgeführt sein. Bei der in Fig. 2 gezeigten Ausführungsform ist der Kohlenstoffdioxidabsorber 1 als Mehrwegkartusche ausgeführt. Durch Betätigen eines Verschlusselements 19 kann der Kohlenstoffdioxidabsorber 1 geöffnet werden, wobei in dem gezeigten Fall das Gehäuse 7 in zwei Teile zerlegt wird. Hierbei wird das Deckelelement 21 mit dem Fensterelement 8 und einigen am Deckelelement 21 befestigten, in geschlossenem Zustand im Strömungskanal 5 angeordneten Bauteilen, hier ein Federelement 22 und eine Druckeinheit 10 mit Anzeigeelement 9, abgenommen. Auf diese Weise wird eine Füllöffnung 20 freigegeben, deren Größe dem Außendurchmesser des Strömungskanals 5 im Bereich der Trennstelle des Gehäuses 7 entspricht und durch die kohlendioxidabsorbierendes Material 6, insbesondere in gesättigtem Zustand, dem Strömungskanal 5 entnommen und neues, ungesättigtes Material 6 in den Strömungskanal eingebracht werden kann.

Der gezeigte Kohlenstoffdioxidabsorber 1 verfügt über einen Einlass 3 und einen Auslass 4, die mit dem Atemgaskreislauf eines Kreislaufatemgerätes 2, wie es beispielsweise in Fig. 1 gezeigt ist, zumindest weitgehend gasdicht verbindbar sind. Der Einlass 3 und der Auslass 4 sind über den Strömungskanal 5 verbunden, in dem kohlenstoffdioxidabsorbierendes Material 6, der sogenannte Atemkalk, in Form eines granulatartigen Schüttgutes als Absorbermaterial angeordnet ist. Als kohlenstoffdioxidabsorbierendes Material 6 wird beispielsweise eine Mischung aus Calciumhydroxid (Ca(OH)₂) und Natriumhydroxid (NaOH) oder aus Kaliumhydroxid (KOH) und Bariumhydroxid (Ba(OH)₂) verwendet.

Im Inneren des Strömungskanals 5 ist eine scheibenförmige Druckeinheit 10 bewegbar angeordnet, wobei diese mithilfe eines Federelements 22, das zwei Spiralfedern aufweist, gegen das Absorbermaterial 6 gedrückt wird. Auf der bewegbar im Strömungskanal 5 angeordneten Druckeinheit 10 ist ein Anzeigeelement 9 in Form eines Quaders, der ein rot eingefärbtes, opakes Kunststoffmaterial aufweist, befestigt. In Abhängigkeit der Füllmenge des innerhalb des Strömungskanals 5 angeordneten Absorbermaterials 6 variiert der Abstand zwischen dem Anzeigeelement 9 und dem Fensterelement 8, das in dem den Strömungskanal 5 umgebenden Gehäuse 7 angeordnet ist, wobei bei einer geringeren Füllmenge der Abstand größer wird.

Das in Fig. 2 dargestellte Fensterelement 8 ist derart in der Wand des Gehäuses 7 angeordnet, dass es von außen aus einer Umgebung des Kohlenstoffdioxidabsorbers 1 den Blick auf das Anzeigeelement 9 freigibt. Durch eine visuelle Überprüfung, also den Blick durch das Fensterelement 8, ist es somit möglich, den Füllstand des Kohlenstoffdioxidabsorbers 1 zu überprüfen, ohne dass dieser hierfür geöffnet werden muss. Einem Benutzer ist es somit auf einfache Weise möglich, insbesondere vor Inbetriebnahme des Kreislaufatemgeräts 2, den Füllstand zu überprüfen. Erfindungswesentlich ist in diesem Zusammenhang, dass der Abstand zwischen dem Fensterelement 8 und dem Anzeigeelement 9 in Abhängigkeit des Füllstandes variiert, sodass sich die Größe der im Fensterelement 8 sichtbaren Abbildung des Anzeigeelements 9 ebenfalls ändert. Je kleiner die sichtbare Abbildung ist, desto geringer ist im Kohlenstoffdioxidabsorber 1 der Füllstand des kohlenstoffdioxidabsorbierenden Materials 6 und damit das Vermögen des Materials 6 Kohlenstoffdioxid zu absorbieren. Im Extremfall, dass keine Abbildung des Anzeigeelements 9 im Fensterelement zu erkennen ist, kann der Nutzer davon ausgehen, dass in dem Kohlenstoffdioxidabsorber 1 nicht mehr genug kohlenstoffabsorbierendes Material 6 für einen sicheren Betrieb enthalten ist.

Während des Betriebs des in Fig. 2 dargestellten Kohlenstoffdioxidabsorbers 1, beispielsweise in einem Kreislauftauchgerät, strömt ausgeatmete Atemluft durch den Einlass 3 in den Kohlenstoffdioxidabsorber 1 ein. Anschließend durchströmt das vom Geräteträger ausgeatmete, mit Kohlenstoffdioxid angereicherte Atemgas das im Strömungskanal 5 des Kohlenstoffdioxidabsorbers 1 angeordnete Absorbermaterial 6, das in diesem Fall aus einer Mischung von Calciumhydroxid (Ca(OH)₂) und Natriumhydroxid (NaOH) gebildet wird, wobei folgende Reaktionen ablaufen:

CO₂ + H₂O <-> H₂CO₃

H₂CO₃ + 2 NaOH <-> Na₂CO₃ + H₂O

Na₂CO₃ + Ca(OH)₂ <-> CaCOs + 2 NaOH

Hierbei wird dem Atemgasstrom Kohlenstoffdioxid unter Bildung von Wasser und Wärme entzogen. Bei fortlaufendem Betrieb eines Kreislaufatemgeräts 2 mit dem darin angeordneten Kohlenstoffdioxidabsorber 1 verringert sich der Füllstand des Absorbermaterials 6. Aufgrund dieser Füllstandsänderung bewegt sich die Druckeinheit 10, die federkraftbeaufschlagt gegen das Absorbermaterial gedrückt wird, nach rechts, wodurch sich der Abstand zwischen dem Fensterelement 8 und dem Anzeigeelement 9 vergrößert. Hierzu zeigt die Detailansicht "A" eine Draufsicht auf das Fensterelement 8, auf dem sich eine Markierung 11 mit mehreren konzentrischen Kreisen befindet und im dem eine mittig ausgerichtete Abbildung des Anzeigeelements 9 zu sehen ist. Je größer der Abstand zwischen dem Fensterelement 8 und dem Anzeigeelement wird, desto kleiner wird die Abbildung des Anzeigeelements 9 innerhalb des Fensterelements 8. Aufgrund der auf dem Fensterelement 8 vorgesehenen Markierung 11 mit konzentrischen Kreisen, deren Radius jeweils in Abhängigkeit eines bestimmten Abstandes zwischen Fensterelement 8 und Anzeigeelement gewählt wurde, kann eine Änderung des Abstandes und damit des Füllstandes von kohlenstoffdioxidabsorbierendem Material 6 von einem Nutzer schnell und genau erfasst werden.

Von besonderem Vorteil ist es, wenn auch ein Kreislaufatemgerät, in dem der in Fig. 2 dargestellte Kohlenstoffdioxidabsorber angeordnet ist, über ein Sichtfenster verfügt, durch das ein Nutzer, insbesondere der Geräteträger, von außen aus einer Umgebung des Geräts auf das Fensterelement 8 des Kohlenstoffdioxidabsorbers 1 sehen und somit die Abbildung des Anzeigeelements 9 erkennen kann, ohne das Kreislaufatemgerät 2 öffnen zu müssen. In diesem Fall ist es somit nicht erforderlich, das Kreislaufatemgerät 2 zu öffnen, um den Füllstand des jeweils verbauten Kohlenstoffdioxidabsorbers zu überprüfen.

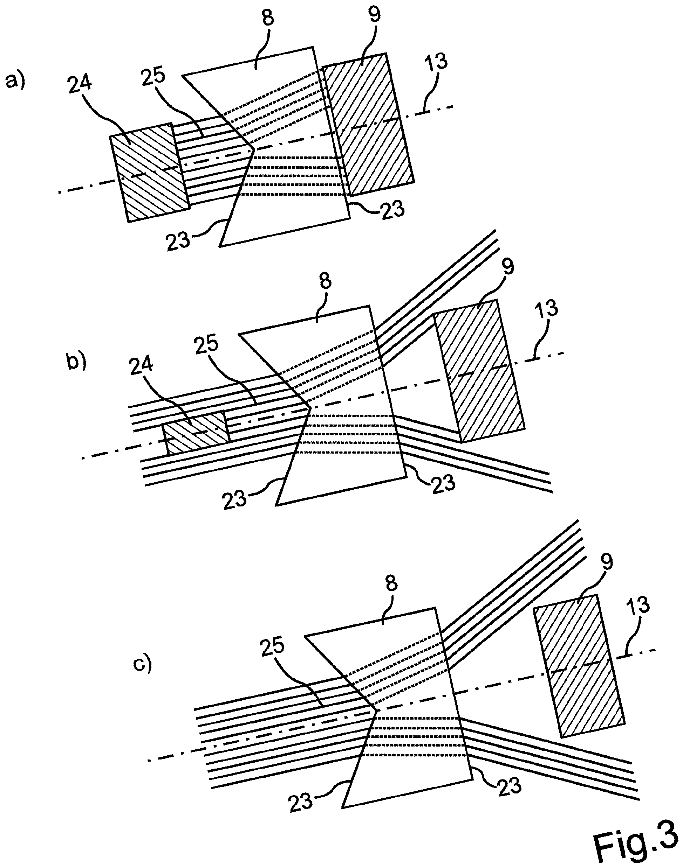

Das Fensterelement 8 ist derart ausgeführt, dass ein Optikelement 12 in dieses integriert ist, das lichtbrechende Eigenschaften aufweist, wobei die Lichtstrahlen jeweils an den Kanten des Fensterelements 8, an denen unterschiedliche Medien aneinandergrenzen, gebrochen werden. Das Optikelement 12 ist derart ausgeführt, dass von außen aus einer Umgebung auf das Fensterelement 8 auftreffende Lichtstrahlen nach dem Durchtritt durch das in das Fensterelement 8 integrierte Optikelement 12 divergent von einer Mittenachse 13, die vom Fensterelement 8 zum Anzeigeelement 9 verläuft, weggelenkt werden. Das hierbei zum Einsatz kommende Wirkprinzip ist in Fig. 3 dargestellt.

Treffen Lichtstrahlen 25 von außer aus der Umgebung auf das Fensterelement 8 mit dem Optikelement 12, werden die Lichtstrahlen 25 an der dem Anzeigeelement 9 zugewandten Kante 23 beim Übergang vom optisch dichteren zum optisch dünneren Medium derart gebrochen, dass die Lichtstrahlen 25 divergent von einer zwischen Fensterelement 8 und Anzeigeelement 9 verlaufen verlaufenden Mittenachse 13 weggelenkt werden. In diesem Zusammenhang zeigt Fig. 3a in einer schematischen Schnittansicht das Fensterelement 8, das hierzu beabstandet im Innenraum des Kohlenstoffdioxidabsorbers 1 angeordnete Anzeigeelement 9 sowie eine Abbildung 24 des Anzeigeelementes 9, die bei einer Betrachtung des Fensterelements 8 erkennbar ist. Die in Fig. 3a gezeigte Abbildung 24 des Anzeigeelements 9 ist bei zumindest nahezu maximalem Füllstand und somit minimalem Abstand zwischen Fensterelement 8 und Anzeigeelement 9 wahrnehmbar.

Wie bereits erläutert, verändert sich der Abstand des Anzeigeelements 9 vom Fensterelement 8 in Abhängigkeit der Füllmenge des innerhalb des Kohlenstoffdioxidabsorbers 1 angeordneten kohlenstoffdioxidabsorbierenden Materials 6. Figur 3B zeigt hierzu einen Betriebszustand, in dem der Füllstand des Absorbermaterials 6 im Vergleich zu dem in Figur 3a gezeigten Betriebszustand abgenommen hat. Es ist zu erkennen ist, dass die im Fensterelement sichtbare Abbildung 24 des Anzeigeelements 9 deutlich kleiner als die in Figur 3a dargestellte Abbildung ist. Dies ist darauf zurückzuführen, dass ein Teil der in das Innere des Kohlenstoffdioxidabsorbers eingestrahlten Lichtstrahlen 25, die divergent von einer zwischen dem Fensterelement 8 und dem Anzeigeelement 9 verlaufenden Mittenachse 13 weggelenkt werden, nicht auf das Anzeigeelement 9 auftreffen und somit auch reflektiert werden. Ein Betrachter, der in das Fensterelement 8 sieht, wird wahrnehmen, dass die Größe der Abbildung 24 des Anzeigeelements 9 im Vergleich zu der Abbildung gemäß Figur 3a deutlich abgenommen hat und wird hieraus auf eine in der Zwischenzeit zwischen den Betriebszuständen gemäß Fig. 3a und Fig. 3b erfolgte Verringerung des Füllstandes des kohlenstoffdioxidabsorbierenden Materials 6 im Kohlenstoffdioxidabsorber 1 schließen.

Fig. 3c zeigt einen weiteren Betriebszustand, bei dem der Füllstand des im Kohlenstoffdioxidabsorbers 1 angeordneten Absorbermaterials 6 im Vergleich zu dem Betriebszustand gemäß Fig. 3b geringer ist oder eventuell sogar kein Atemkalk im Kohlenstoffdioxidabsorber 1 enthalten ist. Der Abstand zwischen dem Fensterelement 8 und dem Anzeigeelement 9 hat sich daher ebenfalls weiter vergrößert. Nunmehr ist der Abstand zwischen dem Fensterelement 8 und dem Anzeigeelement 9 derart groß, dass in das Fensterelement 8 einfallende Lichtstrahlen 25, die divergent von der zwischen dem Fensterelement 8 und dem Anzeigeelement 9 verlaufenden Mittenachse weggelenkt werden, vollständig an dem Anzeigeelement vorbeistrahlen, sodass keine Strahlung von dem Anzeigeelement 9 in Richtung auf das Fensterelement 8 reflektiert wird. Im Fensterelement 8 erscheint somit keine Abbildung des Anzeigeelementes 9, sodass ein das Fensterelement 8 betrachtender Nutzer die Information erhält, dass der Füllstand des Absorbermaterials 6 innerhalb des Kohlenstoffdioxidabsorbers 1 so gering ist, dass neues Absorbermaterial 6 nachgefüllt oder ein anderer Kohlenstoffdioxidabsorber 1 verwendet werden muss.

Wesentlich ist, dass es aufgrund der erfindungsgemäß vorgesehenen Anzeige nicht erforderlich ist, ein Kohlenstoffdioxidabsorber 1 oder eventuell ein Kreislaufatemgerät 2 zu öffnen, um Informationen über den Füllstand des Absorbermaterials 6 zu erhalten.

### Bezugszeichenliste

- 1: Kohlenstoffdioxidabsorber
- 2: Kreislaufatemgerät
- 3: Einlass
- 4: Auslass
- 5: Strömungskanal
- 6: kohlenstoffdioxidabsorbierendes Material
- 7: Gehäuse
- 8: Fensterelement
- 9: Anzeigeelement
- 10: Druckeinheit
- 11: Markierung
- 12: Optikelement
- 13: Mittenachse
- 14: Atemschlauch
14a Ausatemschlauch
14b Einatemschlauch

- 15: Gehäuse des Kreislaufatemgeräts
- 16: Atembeutel
- 17: Sauerstoffquelle
- 18: Atemgaskühler
- 19: Verschlusselement
- 20: Füllöffnung
- 21: Deckelelement
- 22: Federelement
- 23: Kante des Fensterelements
- 24: Abbildung des Anzeigeelements
- 25: Lichtstrahlen

## Patentansprüche

1. Kohlenstoffdioxidabsorber (1) mit einem Einlass (3) und einem Auslass (4), die mit einem Kreislaufatemgerät (2) verbindbar sind und die durch einen Strömungskanal (5) gasdicht verbunden sind, in dem ein Material (6) angeordnet ist, das wenigstens einen Teil des in einem durch das Material (6) geleiteten Atemgasstrom enthaltenen Kohlenstoffdioxids absorbiert,
**dadurch gekennzeichnet, dass** der Strömungskanal (5) wenigstens bereichsweise von einem Gehäuse (7) umgeben ist, in dem ein Fensterelement (8) angeordnet ist, durch das ein im Strömungskanal (5) bewegbar angeordnetes Anzeigeelement (9) von außerhalb des Gehäuses (7) sichtbar ist und/oder durch das vom Anzeigeelement (9) reflektierte Strahlung nach außen tritt, wobei der Kohlenstoffdioxidabsorber (1) so konfiguriert ist, dass ein Abstand zwischen dem Fensterelement (8) und dem Anzeigeelement (9) in Abhängigkeit der im Strömungskanal (5) angeordneten Menge von kohlenstoffdioxidabsorbierendem Material (6) variiert.

2. Kohlenstoffdioxidabsorber nach Anspruch 1,
**dadurch gekennzeichnet, dass** das Anzeigeelement (9) auf einer innerhalb des Strömungskanals (5) bewegbaren Druckeinheit (10) angeordnet ist, die mittelbar oder unmittelbar am Material (6) anliegt.

3. Kohlenstoffdioxidabsorber nach Anspruch 1 oder 2,
**dadurch gekennzeichnet, dass** das Anzeigeelement (9) auf einer Druckeinheit (10) angeordnet ist, die mit einer Federkraft beaufschlagt in Richtung des Materials (6) gedrückt und/oder gezogen wird.

4. Kohlenstoffdioxidabsorber nach Anspruch 2 oder 3,
**dadurch gekennzeichnet, dass** die Druckeinheit (10) wenigstens bereichsweise siebförmig ausgeführt ist.

5. Kohlenstoffdioxidabsorber nach einem der vorangehenden Ansprüche, **dadurch gekennzeichnet, dass** auf dem Fensterelement (8) eine Markierung (11) angeordnet ist.

6. Kohlenstoffdioxidabsorber nach einem der vorangehenden Ansprüche, **dadurch gekennzeichnet, dass** das Anzeigeelement (9) wenigstens ein opakes Material aufweist.

7. Kohlenstoffdioxidabsorber nach einem der vorangehenden Ansprüche, **dadurch gekennzeichnet, dass** ein Optikelement (12) vorgesehen ist, durch das ein von außen aus einer Umgebung in das Fensterelement (8) eintretender Lichtstrahl von einer vom Fensterelement (8) zum Anzeigeelement (9) verlaufenden Mittenachse (13) divergent weggelenkt wird.

8. Kohlenstoffdioxidabsorber nach Anspruch 7,
**dadurch gekennzeichnet, dass** das Optikelement (12) eine Linse und/oder ein Prisma aufweist.

9. Kohlenstoffdioxidabsorber nach Anspruch 7 oder 8,
**dadurch gekennzeichnet, dass** das Optikelement (12) in das Fensterelement integriert ist.

10. Kohlenstoffdioxidabsorber nach einem der vorangehenden Ansprüche, **dadurch gekennzeichnet, dass** das Material (6) als Schüttgut ausgeführt ist.

11. Kohlenstoffdioxidabsorber nach einem der vorangehenden Ansprüche, **dadurch gekennzeichnet, dass** das Material (6) Calciumhydroxid (Ca(OH)₂) und/oder Natriumhydroxid (NaOH) aufweist.

12. Kohlenstoffdioxidabsorber nach einem der vorangehenden Ansprüche, **dadurch gekennzeichnet, dass** das Gehäuse (7) Teil einer Einweg-Kartusche ist.

13. Kohlenstoffdioxidabsorber nach einem der vorangehenden Ansprüche, **dadurch gekennzeichnet, dass** der Strömungskanal (5) wenigstens eine verschließbare Füllöffnung (20) aufweist, durch die das Material (6) wenigstens zeitweise einfüllbar und/oder entnehmbar ist.

14. Kreislaufatemgerät (2), insbesondere Kreislauftauchgerät, mit einem Kohlenstoffdioxidabsorber (1) nach einem der vorangehenden Ansprüche.

## Claims

1. Carbon dioxide absorber (1) having an inlet (3) and an outlet (4) which are connectable to a rebreather apparatus (2) and which are connected in a gas-tight manner by a flow channel (5) in which there is arranged a material (6) which absorbs at least a part of the carbon dioxide present in a respiratory gas flow conducted through the material (6),
**characterized in that** the flow channel (5) is surrounded, at least in certain regions, by a housing (7) in which there is arranged a window element (8) through which a display element (9) movably arranged in the flow channel (5) is visible from outside the housing (7) and/or through which radiation reflected by the display element (9) escapes to the outside, wherein the carbon dioxide absorber (1) is configured such that a distance between the window element (8) and the display element (9) varies in dependence on the quantity of carbon dioxide-absorbing material (6) arranged in the flow channel (5).

2. Carbon dioxide absorber according to Claim 1,
**characterized in that** the display element (9) is arranged on a pressure unit (10) which is movable within the flow channel (5) and which bears indirectly or directly against the material (6).

3. Carbon dioxide absorber according to Claim 1 or 2,
**characterized in that** the display element (9) is arranged on a pressure unit (10) which is pushed and/or pulled in the direction of the material (6) under the action of a spring force.

4. Carbon dioxide absorber according to Claim 2 or 3,
**characterized in that** the pressure unit (10) is embodied, at least in certain regions, in a screen-like manner.

5. Carbon dioxide absorber according to one of the preceding claims, **characterized in that** a marking (11) is arranged on the window element (8).

6. Carbon dioxide absorber according to one of the preceding claims, **characterized in that** the display element (9) comprises at least one opaque material.

7. Carbon dioxide absorber according to one of the preceding claims, **characterized in that** an optical element (12) is provided by means of which a light beam entering the window element (8) from the outside from a surrounding area is deflected away so as to diverge from a central axis (13) running from the window element (8) to the display element (9).

8. Carbon dioxide absorber according to Claim 7,
**characterized in that** the optical element (12) is a lens and/or a prism.

9. Carbon dioxide absorber according to Claim 7 or 8,
**characterized in that** the optical element (12) is integrated into the window element.

10. Carbon dioxide absorber according to one of the preceding claims, **characterized in that** the material (6) is embodied as bulk material.

11. Carbon dioxide absorber according to one of the preceding claims, **characterized in that** the material (6) comprises calcium hydroxide (Ca(OH)₂) and/or sodium hydroxide (NaOH).

12. Carbon dioxide absorber according to one of the preceding claims, **characterized in that** the housing (7) is part of a single-use cartridge.

13. Carbon dioxide absorber according to one of the preceding claims, **characterized in that** the flow channel (5) has at least one closable filling opening (20) through which the material (6) can at least intermittently be introduced and/or removed.

14. Rebreather apparatus (2), in particular diving rebreather apparatus, having a carbon dioxide absorber (1) according to one of the preceding claims.

## Revendications

1. Absorbeur de dioxyde de carbone (1) comportant une entrée (3) et une sortie (4) qui peuvent être reliées à un appareil respiratoire à circuit fermé (2) et qui sont reliées de manière étanche au gaz par un canal d'écoulement (5) dans lequel est disposé un matériau (6) qui absorbe au moins une partie du dioxyde de carbone contenu dans un flux de gaz respiratoire conduit à travers le matériau (6),
**caractérisé en ce que** le canal d'écoulement (5) est entouré au moins dans certaines zones par un boîtier (7) dans lequel est disposé un élément formant fenêtre (8) à travers lequel un élément indicateur (9) disposé de manière mobile dans le canal d'écoulement (5) est visible depuis l'extérieur du boîtier (7) et/ou à travers lequel le rayonnement réfléchi par l'élément indicateur (9) sort vers l'extérieur, l'absorbeur de dioxyde de carbone (1) étant configuré de telle sorte qu'une distance entre l'élément formant fenêtre (8) et l'élément indicateur (9) varie en fonction de la quantité de matériau (6) absorbant le dioxyde de carbone disposée dans le canal d'écoulement (5).

2. Absorbeur de dioxyde de carbone selon la revendication 1,
**caractérisé en ce que** l'élément indicateur (9) est disposé sur une unité de pression (10) mobile à l'intérieur du canal d'écoulement (5), qui est en contact direct ou indirect avec le matériau (6).

3. Absorbeur de dioxyde de carbone selon la revendication 1 ou 2,
**caractérisé en ce que** l'élément indicateur (9) est disposé sur une unité de pression (10) qui est pressée et/ou tirée en direction du matériau (6) sous l'effet d'une force élastique.

4. Absorbeur de dioxyde de carbone selon la revendication 2 ou 3,
**caractérisé en ce que** l'unité de pression (10) est réalisée au moins dans certaines zones en forme de tamis.

5. Absorbeur de dioxyde de carbone selon l'une des revendications précédentes, **caractérisé en ce qu'**un marquage (11) est disposé sur l'élément formant fenêtre (8).

6. Absorbeur de dioxyde de carbone selon l'une des revendications précédentes, **caractérisé en ce que** l'élément indicateur (9) présente au moins un matériau opaque.

7. Absorbeur de dioxyde de carbone selon l'une des revendications précédentes, **caractérisé en ce qu'**il est prévu un élément optique (12) par lequel un rayon lumineux pénétrant dans l'élément formant fenêtre (8) depuis l'extérieur en provenance d'un environnement est dévié de manière divergente par rapport à un axe central (13) s'étendant de l'élément formant fenêtre (8) à l'élément indicateur (9).

8. Absorbeur de dioxyde de carbone selon la revendication 7,
**caractérisé en ce que** l'élément optique (12) présente une lentille et/ou un prisme.

9. Absorbeur de dioxyde de carbone selon la revendication 7 ou 8,
**caractérisé en ce que** l'élément optique (12) est intégré dans l'élément formant fenêtre.

10. Absorbeur de dioxyde de carbone selon l'une des revendications précédentes, **caractérisé en ce que** le matériau (6) est réalisé sous forme de produit en vrac.

11. Absorbeur de dioxyde de carbone selon l'une des revendications précédentes, **caractérisé en ce que** le matériau (6) présente de l'hydroxyde de calcium (Ca(OH)₂) et/ou de l'hydroxyde de sodium (NaOH).

12. Absorbeur de dioxyde de carbone selon l'une des revendications précédentes, **caractérisé en ce que** le boîtier (7) fait partie d'une cartouche à usage unique.

13. Absorbeur de dioxyde de carbone selon l'une des revendications précédentes, **caractérisé en ce que** le canal d'écoulement (5) présente au moins une ouverture de remplissage (20) pouvant être fermée, à travers laquelle il est possible d'introduire et/ou de retirer le matériau (6) au moins temporairement.

14. Appareil respiratoire à circuit fermé (2), en particulier appareil de plongée à circuit fermé, doté d'un absorbeur de dioxyde de carbone (1) selon l'une des revendications précédentes.
